# EUROPÄISCHE PATENTSCHRIFT

(11) **EP 1 066 244 B1**
(45) Veröffentlichungstag und Bekanntmachung des Hinweises auf die Patenterteilung: **09.07.2003**
(21) Anmeldenummer: 99914538.6
(22) Anmeldetag: 25.03.1999
(51) Int. Cl.: C07C 231/12

(54) **VERFAHREN ZUR HERSTELLUNG VON 2,4-DICHLOR-5-HYDROXYACETANILID**
METHOD FOR PRODUCING 2,4-DICHLORO-5-HYDROXYACETANILIDE
PROCEDE DE PREPARATION DE 2,4-DICHLORO-5-HYDROXYACETANILIDE

(30) Priorität: 27.03.1998 DE 19813886
(43) Veröffentlichungstag der Anmeldung: 10.01.2001
(73) Patentinhaber: Clariant GmbH, 65929 Frankfurt am Main (DE)
(72) Erfinder: SISTIG, Frank, D-65510 Idstein (DE); LEITUNG, Hans-Jürgen, D-65933 Frankfurt (DE); KRAUSE, Stefan, D-65929 Frankfurt (DE)
(86) Internationale Anmeldenummer: EP9902017
(87) Internationale Veröffentlichungsnummer: WO99050228

(56) Entgegenhaltungen:
- WO-A-85/01939
- WO-A-86/00072
- PATENT ABSTRACTS OF JAPAN vol. 010, no. 203 (C-360), 16. Juli 1986 & JP 61 044856 A (OSAKA SODA CO LTD), 4. März 1986
- PATENT ABSTRACTS OF JAPAN vol. 010, no. 208 (C-361), 22. Juli 1986 & JP 61 047450 A (OSAKA SODA CO LTD), 7. März 1986
- W. A. JACOBS ET AL.: "On certain aromatic amines and chloroacetyl derivatives." JOURNAL OF THE AMERICAN CHEMICAL SOCIETY., Bd. 41, 1919, Seiten 458-474, XP002106321 DC US

## Beschreibung

Die vorliegende Erfindung betrifft ein gegenüber dem Stand der Technik verbessertes Verfahren zur Herstellung von 2,4-Dichlor-5-hydroxyacetanilid.

Das 2,4-Dichlor-5-hydroxyacetanilid dient als wichtige Vorstufe zur Herstellung von Pflanzenschutzmitteln (WO 85/01939).

2,4-Dichlor-5-hydroxyacetanilid läßt sich durch Chlorierung von 3-Hydroxyacetanilid, das durch Acetylierung von 3-Aminophenol zugänglich ist, herstellen.

Die in der WO 86/00072 auf Seite 25 unter Example III Step B beschriebene Umsetzung von 3-Hydroxyacetanilid mit Chlorgas in Eisessig führt zu einer Ausbeute, bezogen auf eingesetztes 3-Hydroxyacetanilid, von lediglich 30,4 % der Theorie.

Die WO 85/01939 beschreibt auf Seite 27 unter Example 3 Step B unter Bezugnahme auf das Example 2 Step B die Umsetzung von 3-Hydroxyacetanilid mit Sulfurylchlorid in Eisessig. Gemäß den Angaben auf Seite 25 Example 2 Step B läßt man bei der Herstellung von 2,4-Dichlor-5-methylacetanilid nach Zugabe des Sulfurylchlorids zu 3-Acetamidotoluol die Reaktionsmischung bei Raumtemperatur ungefähr 60 Stunden stehen, erhitzt anschließend bis sich eine rührfähige Masse bildet und rührt nochmals ungefähr 5 Stunden. Das gemäß Example 3 Step B auf analoge Weise zu Example 2 Step B hergestellte 2,4-Dichlor-5-hydroxyacetanilid fällt in einer Ausbeute von 43,5 %, bezogen auf eingesetztes 3-Hydroxyacetanilid, an.

Die in den beiden vorstehend beschriebenen Beispielen erzielten Ausbeuten an 2,4-Dichlor-5-hydroxyacetanilid sind vergleichsweise niedrig und die Verfahren somit für eine technische Realisierung nicht zu empfehlen.

Es bestand daher die Aufgabe, ein Verfahren zu entwickeln, das sich zum einen auf einfache Art und Weise technisch realisieren läßt, und zudem mit vertretbarem Aufwand das 2,4-Dichlor-5-hydroxyacetanilid in hohen Ausbeuten und zugleich hoher Reinheit zugänglich macht.

Diese Aufgabe wird gelöst durch ein Verfahren zur Herstellung von 2,4-Dichlor-5-hydroxyacetanilid durch Umsetzung von 3-Hydroxyacetanilid mit Sulfurylchlorid in Anwesenheit von 3 bis 30 Gewichtsteilen einer aliphatischen Carbonsäure mit 1 bis 6 Kohlenstoffatomen je Gewichtsteil 3-Hydroxyacetanilid unter intensiver Vermischung bei 20 bis 100°C, Abtrennung gasförmiger Bestandteile aus dem Reaktionsgemisch, Einstellen eines Verhältnisses von 1,0 bis 6 Gewichtsteilen aliphatische Carbonsäure je Gewichtsteil eingesetztes 3-Hydroxyacetanilid und Abtrennen von 2,4-Dichlor-5-hydroxyacetanilid als Feststoff.

Im Hinblick auf die in der WO 85/01939 unter Example 3 Step B beschriebene Herstellung von 2,4-Dichlor-5-hydroxyacetanilid ist es als sehr überraschend anzusehen, daß das erfindungsgemäße Verfahren das gewünschte 2,4-Dichlor-5-hydroxyacetanilid in einer Ausbeute von 80 bis 85 % und mehr, bezogen auf das zur Herstellung von 3-Hydroxyacetanilid benötigte 3-Aminophenol, zugänglich macht. Das Verfahren läßt sich ohne besonderen technischen Aufwand bereits bei vergleichsweise niedrigen Temperaturen und kurzen Reaktionszeiten realisieren. Die im Verlauf der erfindungsgemäßen Arbeitsweise abgetrennte Essigsäure kann wiederum als Lösungsmittel/Suspensionsmittel in die Umsetzung eingesetzt werden.

Man setzt je Mol 3-Hydroxyacetanilid üblicherweise 1,5 bis 5, insbesondere 1,8 bis 2,5, bevorzugt 1,9 bis 2,2 Mol Sulfurylchlorid ein.

Man setzt die aliphatische Carbonsäure, bezogen auf 3-Hydroxyacetanilid, in einer solchen Menge zu, daß während der Umsetzung eine ausreichende Durchmischung des Reaktionsgemisches gewährleistet ist.

in einer Vielzahl von Fällen ist dies sichergestellt, indem man die Umsetzung von 3-Hydroxyacetanilid mit Sulfurylchlorid in Anwesenheit von 4 bis 15, insbesondere 4,5 bis 13, bevorzugt 5 bis 10, besonders bevorzugt 5 bis 7 Gewichtsteilen aliphatischer Carbonsäure je Gewichtsteil 3-Hydroxyacetanilid durchführt.

Als aliphatische Carbonsäure eignen sich Ameisensäure, Essigsäure, Propionsäure, n-Buttersäure, i-Buttersäure, 3-Methylbuttersäure, Valeriansäure, n-Hexan und i-Hexansäure. Es lassen sich auch Mischungen der vorstehend genannten Carbonsäuren verwenden. Man verwendet die aliphatischen Carbonsäuren respektive deren Mischungen in wasserfreier Form.

Als aliphatische Carbonsäure eignen sich besonders gut Ameisensäure, Essigsäure, Propionsäure, n-Buttersäure, i-Buttersäure oder Mischungen dieser Carbonsäuren, insbesondere Ameisensäure, Essigsäure oder Propionsäure, bevorzugt Essigsäure.

In einer Vielzahl von Fällen hat es sich als vorteilhaft erwiesen, 3-Hydroxy-acetanilid mit Sulfurylchlorid bei 25 bis 70, insbesondere 40 bis 60°C umzusetzen.

Besonders einfach gestaltet sich das Verfahren, in dem man das 3-Hydroxyacetanilid und aliphatische Carbonsäure vorlegt und das Sulfurylchlorid unter Vermischung zusetzt. Die Zugabegeschwindigkeit des Sulfurylchlorids wird so gewählt, daß die Reaktionswärme und die sich bildenden gasförmigen Bestandteile (SO₂, HCI) abgeführt werden können. Die Reaktionszeit ergibt sich im wesentlichen aus der Zugabegeschwindigkeit des Sulfurylchlorids und der Möglichkeit, sowohl die Reaktionswärme als auch die gasförmigen Bestandteile (Abgase) in ausreichender Geschwindigkeit abführen zu können.

Während der Umsetzung ist dafür Sorge zu tragen, daß aus dem Reaktionsgemisch gasförmige Bestandteile entweichen können. Nach Beendigung der Zugabe von Sulfurylchlorid läßt man noch eine ausreichende Zeit nachreagieren, um die Umsetzung zu vervollständigen und eventuell noch vorhandene gasförmige Bestandteile zu entfernen.

Während der Umsetzung ist zu gewährleisten, daß die Reaktanten gut durchmischt werden. Eine gute Durchmischung begünstigt auch die erforderliche Abtrennung gasförmiger Bestandteile aus dem Reaktionsgemisch während der Umsetzung und Nachreaktion.

Die nach der Umsetzung mit Sulfurylchlorid noch gelösten gasförmigen Bestandteile, beispielsweise HCI und SO₂, trennt man üblicherweise bei einem Druck von 10 mbar bis Atmosphärendruck, insbesondere 20 mbar bis 500 mbar, bevorzugt 50 bis 250 mbar ab. Es ist dafür Sorge zu tragen, daß die gasförmigen Bestandteile möglichst weitgehend aus dem Reaktionsgemisch abgetrennt werden. Eine unzureichende Abtrennung der gasförmigen Bestandteile aus dem Reaktionsgemisch kann sich sowohl auf die Ausbeute als auch auf die Reinheit des Endproduktes nachteilig auswirken.

In vielen Fällen hat es sich als vorteilhaft erwiesen, gasförmige Bestandteile bei 25 bis 100°C, insbesondere 40 bis 70°C zu entfernen.

Bei Abtrennung der gasförmigen Bestandteile wählt man üblicherweise Temperatur und Druck dermaßen, daß zwar die gasförmigen Bestandteile entfernt werden, nicht aber die aliphatische Carbonsäure abgetrennt wird. Auf diese Weise wird sichergestellt, daß bei einer destillativen Abtrennung der aliphatischen Carbonsäure, eine nicht durch HCI und SO₂ verunreinigte aliphatische Carbonsäure gewonnen werden kann.

Wie zuvor erwähnt, stellt man das Verhältnis aliphatische Carbonsäure zu eingesetztem 3-Hydroxyacetanilid auf 1,0 bis 6, insbesondere 1,5 bis 5, bevorzugt 1,5 bis 4,5, besonders bevorzugt 2 bis 4, Gewichtsteile aliphatische Carbonsäure je Gewichtsteil eingesetztes 3-Hydroxyacetanilid ein, indem man, falls erforderlich, aus dem Reaktionsgemisch die aliphatische Carbonsäure bis zu dem vorstehend bereits genannten Verhältnis abtrennt oder, falls erforderlich, eine entsprechende Menge aliphatische Carbonsäure zugibt. Auf eine separate Einstellung dieses Verhältnisses kann gegebenenfalls verzichtet werden, falls bei der Umsetzung von 3-Hydroxyacetanilid mit Sulfurylchlorid von vornherein ein für die Abtrennung von 2,4-Dichlor-5-hydroxyacetanilid passendes Verhältnis von aliphatischer Carbonsäure zu eingesetztem 3-Hydroxyacetanilid eingestellt wird.

Das Verhältnis aliphatische Carbonsäure zu eingesetztem 3-Hydroxyacetanilid soll so bemessen sein, daß einerseits das 2,4-Dichlor-5-hydroxyacetanilid möglichst vollständig als Feststoff ausfällt und andererseits das anfallende Reaktionsgemisch eine für die weitere Aufarbeitung (Filtrieren, Zentrifugieren) ausreichende Fließfähigkeit besitzt.

In einer Vielzahl von Fällen hat es sich bewährt, die aliphatische Carbonsäure bei 30 bis 80°C und 25 bis 270 mbar, abzudestillieren. Anschließend kühlt man das Reaktionsgemisch auf 15 bis 45°C ab und trennt das 2,4-Dichlor-5-hydroxyacetanilid als Feststoff, beispielsweise durch Filtration, ab.

Die hierbei anfallende 2,4-Dichlor-5-hydroxyacetanilid enthaltende Mutterlauge kann mit gutem Erfolg zur Erhöhung der Ausbeute in einer Menge von 10 bis 90 %, insbesondere 30 bis 60 % in die nächste Umsetzung mit Sulfurylchlorid zurückgeführt werden. Die Einsatzmengen an frischer aliphatischer Carbonsäure lassen sich dann entsprechend reduzieren.

Das als Feststoff abgetrennte 2,4-Dichlor-5-hydroxyacetanilid fällt nach Waschen mit Essigsäure und Wasser üblicherweise in einer Reinheit von 98,5 % und höher an.

Man kann in das erfindungsgemäße Verfahren sowohl 3-Hydroxyacetanilid in isolierter Form oder auch in Form eines Rohproduktes einsetzen.

Nach einer besonderen Ausführungsform des erfindungsgemäßen Verfahrens setzt man als 3-Hydroxyacetanilid ein durch Umsetzung von 3-Aminophenol mit Acetanhydrid in Essigsäure hergestelltes 3-Hydroxyacetanilid enthaltendes Reaktionsgemisch ein. Diese Variante ist besonders vorteilhaft, da man dieses Reaktionsgemisch ohne zusätzliche Reinigung direkt in das Verfahren einsetzen kann.

Zur Herstellung dieses 3-Hydroxyacetanilid enthaltenden Reaktionsgemisches setzt man 3-Aminophenol in Essigsäure als Lösungsmittel mit 0,9 bis 2, insbesondere 1 bis 1,2 Mol Essigsäureanhydrid um. Dabei hat es sich bewährt, 3-Aminophenol in Essigsäure gelöst vorzulegen und Essigsäureanhydrid langsam zuzugeben. Die Reaktion erfolgt bei einer Temperatur von 0 bis 120°C, insbesondere von 20 bis 60°C. Die Essigsäuremenge wird so gewählt, daß die Reaktionspartner bei Reaktionstemperatur gelöst vorliegen. In vielen Fällen haben sich 1 bis 20, insbesondere 2 bis 5 Gewichtsteile Essigsäure, bezogen auf Gewichtsteil 3-Aminophenol, bewährt. Die Reaktionszeit ergibt sich aus der gewählten Reaktionstemperatur und der Geschwindigkeit, mit der die Reaktionspartner zusammengegeben werden. In vielen Fällen ist eine Reaktionszeit von 30 Minuten bis 10 Stunden, insbesondere von 1 bis 3 Stunden, ausreichend.

Nach erfolgter Umsetzung kann 3-Hyxdroxyacetanilid aus dem Reaktionsgemisch auskristallisiert und abfiltriert werden. Die Kristallisation erfolgt üblicherweise bei 0 bis 40°C, insbesondere bei 15 bis 20°C

Wie zuvor erwähnt, kann sowohl das 3-Hydroxyacetanilid in isolierter Form als auch das 3-Hydroxyacetanilid enthaltende Reaktionsgemisch ohne weitere Reinigung in die Chlorierung mit Sulfurylchlorid eingesetzt werden.

Setzt man ein derartiges Reaktionsgemisch ein, so empfiehlt es sich, entweder durch Zugabe der aliphatischen Carbonsäure, insbesondere von Essigsäure oder durch Abtrennen von Essigsäure das erforderliche Gewichtsverhältnis aliphatische Carbonsäure bzw. Essigsäure zu 3-Hydroxyacetanilid einzustellen. Durch Verwendung von Essigsäure als aliphatische Carbonsäure läßt sich diese Verfahrensvariante besonders günstig durchführen.

Das erfindungsgemäße Verfahren läßt sich sowohl diskontinuierlich als auch kontinuierlich durchführen.

Die nachfolgenden Beispiele beschreiben die Erfindung näher, ohne sie zu beschränken.

### Experimenteller Teil

### A) Herstellung eines 3-Hydroxyacetanilid enthaltenden Reaktionsgemisches

262 g (2,4 Mol) 3-Aminophenol werden bei 20°C in 806 g Essigsäure gelöst. Anschließend werden innerhalb von 45 Minuten 254 g Essigsäureanhydrid zugegeben. Nach erfolgter Zugabe wird 5 Minuten bei 55°C nachgerührt. Wie eine HPLC-Analyse beweist, hat sich 3-Aminophenol vollständig zu 3-Hydroxyacetanilid umgesetzt.
Das Reaktionsgemisch wird direkt und ohne weitere Reinigung in die nachfolgende Chlorierung eingesetzt.

### B) Herstellung von 2,4-Dichlor-5-hydroxyacetanilid

### Beispiel 1: Umsetzung von 3-Hydroxyacetanilid mit Sulfurylchlorid

175 g (1,16 Mol) 3-Hydroxyacetanilid werden in 2700 g Eisessig gelöst. Unter intensivem Rühren werden bei 60°C innerhalb von 2 Stunden 327 g (2,42 Mol) Sulfurylchlorid zugetropft. Nach erfolgter Zugabe wird 5 Minuten bei 60°C nachgerührt. Anschließend werden bei 60°C Sumpftemperatur und ca. 120 mbar zunächst noch gelöste Gase entfernt und dann 2000 g Essigsäure abdestilliert. Die Suspension wird auf 15°C abgekühlt, abfiltriert und der Rückstand mit 250 g Essig und 250 g Wasser gewaschen. Der Filterkuchen wird bei ca. 200 mbar und 60°C getrocknet. Es werden 193 g 2,4-Dichlor-5-hydroxyacetanilid, entsprechend 76 % Ausbeute, erhalten.
Die Reinheit des 2,4-Dichlor-5-hydroxyacetanilids ist - bestimmt mittels HPLC - größer als 99 %.

### Beispiel 2: Umsetzung von 3-Hydroxyacetanilid mit Sulfurylchlorid unter Rückführung der Mutterlauge aus Beispiel 1

175 g (1,16 Mol) 3-Hydroxyacetanilid werden in 2430 g Eisessig und 270 g der aus Beispiel 1 stammenden vereinigten Menge aus Mutterlauge und Waschessig versetzt. Unter intensivem Rühren werden bei 60°C innerhalb von 2 Stunden 327 g Sulfurylchlorid zugetropft. Nach erfolgter Zugabe wird 5 Minuten bei 60°C nachgerührt. Anschließend werden bei 60°C Sumpftemperatur und ca. 120 mbar zunächst noch gelöste Gase entfernt und dann 2000 g Essigsäure abdestilliert.

Die Suspension wird auf 15°C kaltgerührt, abfiltriert und der Rückstand wird mit 250 g Essigsäure und 250 g Wasser gewaschen. Der Filterkuchen wird bei ca. 200 mbar und 60°C getrocknet. Es werden 215 g 2,4-Dichlor-5-hydroxyacetanilid, entsprechend 84 % Ausbeute, erhalten.
Die Reinheit, bestimmt mittels HPLC-Analyse ist größer 99 %.

### Beispiel 3: Umsetzung des gemäß A) hergestellten Reaktionsgemisches mit Sulfurylchlorid

Das Reaktionsgemisch, hergestellt gemäß A), wird mit 2000 g Essigsäure versetzt. Anschließend werden bei 30°C innerhalb von 60 Minuten 665 g Sulfurylchlorid unter intensivem Rühren zugetropft. Nach erfolgter Zugabe des Sulfurylchlorids wird 5 Minuten bei 30°C nachgerührt. Danach werden bei ca. 50°C Sumpftemperatur und einem reduziertem Druck von ca. 100 mbar zunächst noch gelöste Gase entfernt und dann 1500 g Essigsäure abdestilliert. Die anfallende Suspension wird auf 20°C abgekühlt und filtriert. Der dabei anfallende Filterkuchen wird mit 500 g Essigsäure und 500 ml Wasser gewaschen und bei ca. 200 mbar und 60°C getrocknet.

Es werden 425 g 2,4-Dichlor-5-hydroxyacetanilid, entsprechend 80 % Ausbeute, bezogen auf 3-Aminophenol, als weißer Feststoff erhalten.
Die Reinheit, bestimmt durch HPLC-Analyse, ist größer 99 %.

### Beispiel 4: Umsetzung von 3-Hydroxyacetanilid mit Sulfurylchlorid in Ameisensäure

151 g 3-Hydroxyacetanilid werden in 1500 g Ameisensäure gelöst. Unter intensivem Rühren werden bei 60°C innerhalb von 2 Stunden 277 g Sulfurylchlorid zugetropft. Nach beendeter Zugabe wird 5 Minuten bei 60°C nachgerührt. Anschließend werden bei 60°C Sumpftemperatur und ca. 140 mbar zunächst noch gelöste Gase entfernt und dann 1050 g Ameisensäure abdestilliert.

Die Suspension wird auf 15°C kaltgerührt, abfiltriert und der Rückstand mit 200 g Ameisensäure und 200 g Wasser gewaschen. Der Filterkuchen wird bei ca. 200 mbar und 60°C getrocknet. Es werden 154 g 2,4-Dichlor-5-hydroxyacetanilid, entsprechend 70 % Ausbeute, erhalten.
Die Reinheit, bestimmt mittels HPLC-Analyse, ist größer 99 %.

### Beispiel 5: Umsetzung von 3-Hydroxyacetanilid mit Sulfurylchlorid in Propionsäure

Es wird, wie in Beispiel 4 beschrieben, gearbeitet, mit dem Unterschied, daß anstelle von Ameisensäure Propionsäure eingesetzt wird.

Es werden 176 g 2,4-Dichlor-5-hydroxyacetanilid, entsprechend 80 % Ausbeute, erhalten.
Die Reinheit, bestimmt durch HPLC-Analyse, ist größer 99 %.

## Patentansprüche

1. Verfahren zur Herstellung von 2,4-Dichlor-5-hydroxyacetanilid durch Umsetzung von 3-Hydroxyacetanilid mit Sulfurylchlorid in Anwesenheit von 3 bis 30 Gewichtsteilen einer aliphatischen Carbonsäure mit 1 bis 6 Kohlenstoffatomen je Gewichtsteil 3-Hydroxyacetanilid unter intensiver Vermischung bei 20 bis 100°C, Abtrennung gasförmiger Bestandteile aus dem Reaktionsgemisch, Einstellen eines Verhältnisses von 1,0 bis 6 Gewichtsteilen aliphatische Carbonsäure je Gewichtsteil eingesetztes 3-Hydroxyacetanilid und Abtrennen von 2,4-Dichlor-5-hydroxyacetanilid als Feststoff.

2. Verfahren nach Anspruch 1, **dadurch gekennzeichnet, daß** man je Mol 3-Hydroxyacetanilid 1,5 bis 5 Mol Sulfurylchlorid einsetzt.

3. Verfahren nach Anspruch 1 oder 2, **dadurch gekennzeichnet, daß** man je Mol 3-Hydroxyacetanilid 1,8 bis 2,5 Mol Sulfurylchlorid einsetzt.

4. Verfahren nach einem oder mehreren der Ansprüche 1 bis 3, **dadurch gekennzeichnet, daß** man die Umsetzung in Anwesenheit von 4 bis 15 Gewichtsteilen aliphatische Carbonsäure durchführt.

5. Verfahren nach einem oder mehreren der Ansprüche 1 bis 4, **dadurch gekennzeichnet, daß** man Ameisensäure, Essigsäure oder Propionsäure als aliphatische Carbonsäure einsetzt.

6. Verfahren nach einem oder mehreren der Ansprüche 1 bis 5, **dadurch gekennzeichnet, daß** man Essigsäure als aliphatische Carbonsäure einsetzt.

7. Verfahren nach einem oder mehreren der Ansprüche 1 bis 6, **dadurch gekennzeichnet, daß** man 3-Hydroxyacetanilid mit Sulfurylchlorid bei 25 bis 60°C umsetzt.

8. Verfahren nach einem oder mehreren der Ansprüche 1 bis 7, **dadurch gekennzeichnet, daß** man die gasförmigen Bestandteile bei 10 mbar bis Atmosphärendruck abtrennt.

9. Verfahren nach einem oder mehreren der Ansprüche 1 bis 8, **dadurch gekennzeichnet, daß** man als 3-Hydroxyacetanilid ein durch Umsetzung von 3-Aminophenol mit Acetanhydrid in Essigsäure hergestelltes 3-Hydroxyacetanilid enthaltendes Reaktionsgemisch einsetzt.

10. Verfahren nach einem oder mehreren der Ansprüche 1 bis 9, **dadurch gekennzeichnet, daß** man ein Verhältnis von 1,5 bis 5 Gewichtsteilen aliphatische Carbonsäure je Gewichtsteil eingesetztes 3-Hydroxyacetanilid einstellt.

## Claims

1. A process for preparing 2,4-dichloro-5-hydroxyacetanilide by reacting 3-hydroxyacetanilide with sulfuryl chloride in the presence of from 3 to 30 parts by weight of an aliphatic carboxylic acid having from 1 to 6 carbon atoms per part by weight of 3-hydroxyacetanilide with vigorous mixing at from 20 to 100°C, removing gaseous components from the reaction mixture, setting a ratio of from 1.0 to 6 parts by weight of aliphatic carboxylic acid per part by weight of 3-hydroxyacetanilide employed and removing 2,4-dichloro-5-hydroxyacetanilide as a solid.

2. The process as claimed in claim 1, wherein from 1.5 to 5 mol of sulfuryl chloride are employed per mole of 3-hydroxyacetanilide.

3. The process as claimed in claim 1 or 2, wherein from 1.8 to 2.5 mol of sulfuryl chloride are employed per mole of 3-hydroxyacetanilide.

4. The process as claimed in one or more of claims 1 to 3, wherein the reaction is carried out in the presence of from 4 to 15 parts by weight of aliphatic carboxylic acid.

5. The process as claimed in one or more of claims 1 to 4, wherein the aliphatic carboxylic acid used is formic acid, acetic acid or propionic acid.

6. The process as claimed in one or more of claims 1 to 5, wherein the aliphatic carboxylic acid used is acetic acid.

7. The process as claimed in one or more of claims 1 to 6, wherein 3-hydroxyacetanilide is reacted with sulfuryl chloride at from 25 to 60°C.

8. The process as claimed in one or more of claims 1 to 7, wherein the gaseous components are removed at from 10 mbar to atmospheric pressure.

9. The process as claimed in one or more of claims 1 to 8, wherein the 3-hydroxyacetanilide used is a 3-hydroxyacetanilide-containing reaction mixture prepared by reacting 3-aminophenol with acetic anhydride in acetic acid.

10. The process as claimed in one or more of claims 1 to 9, wherein a ratio of from 1.5 to 5 parts by weight of aliphatic carboxylic acid per part by weight of 3-hydroxyacetanilide employed is set.

## Revendications

1. Procédé de préparation de 2,4-dichloro-5-hydroxyacétanilide par la réaction de 3-hydroxyacétanilide avec du chlorure de sulfuryle en présence de 3 à 30 parties poids d'un acide carboxylique aliphatique présentant 1 à 6 atomes de carbone par partie en poids de 3-hydroxyacétanilide en mélangeant intensivement à une température de 20 à 100°C, par séparation des composants gazeux du mélange réactionnel, par établissement d'un rapport de 1,0 à 6 parties en poids d'acide carboxylique aliphatique par partie en poids de 3-hydroxyacétanilide utilisée et par séparation du 2,4-dichloro-5-hydroxyacétanilide en tant que matière solide.

2. Procédé selon la revendication 1 **caractérisé en ce que** l'on emploie par mole de 3-hydroxyacétanilide de 1,5 à 5 moles de chlorure de sulfuryle.

3. Procédé selon la revendication 1 ou 2 **caractérisé en ce que** l'on emploie par mole de 3-hydroxyacétanilide de 1,8 à 2,5 moles de chlorure de sulfuryle.

4. Procédé selon une ou plusieurs des revendications 1 à 3 **caractérisé en ce que** l'on réalise la réaction avec de 4 à 15 parties en poids d'un acide carboxylique aliphatique

5. Procédé selon une ou plusieurs des revendications 1 à 4 **caractérisé en ce que** l'on emploie l'acide formique, l'acide acétique ou l'acide propionique en tant qu'acide carboxylique aliphatique.

6. Procédé selon une ou plusieurs des revendications 1 à 5 **caractérisé en ce que** l'on emploie l'acide acétique en tant qu'acide carboxylique aliphatique.

7. Procédé selon une ou plusieurs des revendications 1 à 6 **caractérisé en ce que** l'on transforme le 3-hydroxyacétanilide avec du chlorure de sulfuryle à une température de 25 à 60°C.

8. Procédé selon une ou plusieurs des revendications 1 à 7 **caractérisé en ce que** l'on sépare les composants gazeux à une pression de 10 mbar et jusqu'à la pression atmosphérique.

9. Procédé selon une ou plusieurs des revendications 1 à 8 **caractérisé en ce que** l'on emploie en tant que 3-hydroxyacétanilide un mélange réactionnel contenant du 3-hydroxyacétanilide préparé par réaction du 3-aminophénol avec de l'anhydride acétique dans de l'acide acétique.

10. Procédé selon une ou plusieurs des revendications 1 à 9 **caractérisé en ce que** l'on établit un rapport de 1,5 à 5 parties en poids d'acide carboxylique aliphatique par partie en poids de 3-hydroxyacétanilide utilisée.
